# EUROPÄISCHE PATENTSCHRIFT

(11) **EP 2 033 675 B1**
(45) Veröffentlichungstag und Bekanntmachung des Hinweises auf die Patenterteilung: **09.07.2014**
(21) Anmeldenummer: 08163652.4
(22) Anmeldetag: 04.09.2008
(51) Int. Cl.: A61M 11/02, A61M 16/10, A61M 16/00

(54) **Inhalationsgerät**
Inhalation device
Appareil d'inhalation

(30) Priorität: 06.09.2007 EP 07115812
(43) Veröffentlichungstag der Anmeldung: 11.03.2009
(73) Patentinhaber: Vectura GmbH, 35285 Gemünden (DE)
(72) Erfinder: Müllinger, Bernhard, 80634, München (DE); Frey, Manuel, 82110, Germering (DE); Kolb, Tobias, 80687, München (DE); Hoffmann, Tobias, 82205 Gilching (DE)
(74) Vertreter: Vossius & Partner

(56) Entgegenhaltungen:
- WO-A-2004/105846
- WO-A-2006/075184
- US-A- 3 817 246
- US-A- 3 842 828
- US-A- 5 237 987

## Beschreibung

Die Erfindung betrifft eine Vorrichtung zum Bereitstellen eines Aersosolflusses und/oder eines Luftflusses und insbesondere ein Inhalationsgerät.

Inhalation, das heißt das therapeutische Einatmen von Nebel, hat sich als eine wirkungsvolle und schonende Methode bei der Behandlung verschiedener Atemwegserkrankungen wie beispielsweise des akuten Atemweginfektes, der chronischen Bronchitis und insbesondere des Asthma bronchiale erwiesen. Dabei bewirkt die Befeuchtung der Schleimhaut mit einem Nebel von feinsten Tröpfchen eine Schleimlösung in den Atemwegen und fördert dadurch das Abhusten von Sekret. Zusätzlich inhalierte Medikamente können gezielt in den Bronchien oder der Lunge appliziert werden, wo sie zur Behandlung einer topischen sowie auch systemischen Erkrankung wirksam sind. Zur Bereitstellung des Inhalationsnebels oder Aerosols wird üblicherweise ein Düsenvernebler eingesetzt, der unter Einsatz eines Kompressors und einer Vernebelungsdüse den Wirkstoff zerstäubt. Die Eindringtiefe der vernebelten Tröpfchen in die Lunge hängt dabei unter anderem von der Tröpfchengröße ab. Ferner lässt sich die gezielte Wirkung der Tröpfchen dadurch steuern, dass nur während einer bestimmten Zeitspanne innerhalb des Einatemvorganges vernebelt wird.

Daher haben sich Inhalationsgeräte durchgesetzt, bei denen sich der Vernebelungsprozess in Abhängigkeit von der Inhalations- bzw. Exhalationsphase regeln lässt. So beschreibt beispielsweise die DE 199 39 417 A1 ein Inhalationsgerät mit einer Steuerungseinrichtung, mit der ein Pneumatikventil in Abhängigkeit von einer Inhalationsphase, einer Exhalationsphase und einer Ruhepause steuerbar ist. Die genannten Phasen können dabei von einem Drucksensor bestimmt werden. Eine weitere automatisierte Inhalationsvorrichtung ist in der EP 1 700 614 A1 offenbart. Darin steuert eine Steuereinrichtung eine Luftpumpe derart über Spannung und/oder über Pulsweitenmodulation an, dass sie einen Inhalationfluss und/oder ein Inhalationsvolumen gemäß einem vorgegebenen zeitlichen Verlauf an einen an die Luftpumpe angeschlossenen Vernebler liefert.

Ein Beatmungsgerät mit einer Verstärkungseinrichtung ist in der US 3,842,828 offenbart.

Generell ist es wünschenswert, dass derartige Inhalationsgeräte möglichst klein ausgebildet sind, da insbesondere chronisch kranke Patienten diese Geräte häufig mit sich führen. Die Größe und das Gewicht derartiger Geräte sind dabei häufig durch den Kompressor vorgegeben. Der Kompressor hat dabei die Aufgabe, einerseits einen hinreichend großen Druck zu erzeugen, um die Vernebelungsdüse zu betreiben, und andererseits einen hinreichend großen Luftfluss bereitzustellen, um dem Atemminutenvolumen des Patienten gerecht zu werden. Hoher Druck und gleichzeitig hohe Flüsse verlangen aber nach einem leistungsstarken Kompressor, der entsprechend voluminös bzw. schwer sein kann.

Es ist demnach eine Aufgabe der vorliegenden Erfindung, eine verbesserte Vorrichtung zum Bereitstellen eines Aerosolflusses und/oder eines Luftflusses bereitzustellen. Diese Aufgabe wird mit den Merkmalen der Ansprüche gelöst.

Der vorliegenden Erfindung liegt die Idee zugrunde, mit einem möglichst kleinen Kompressor einen maximalen Luftfluss bereitzustellen. Dementsprechend betrifft die vorliegende Erfindung eine Vorrichtung zum Bereitstellen eines Aerosolflusses und/oder eines Luftflusses mit mindestens einem Kompressor zum Bereitstellen eines Luftflusses, einer Vernebelungseinrichtung zum Erzeugen eines Aerosolflusses und einer Mischeinrichtung zum optionalen Vermischen des Aerosolflusses mit dem Luftfluss zu einem Gesamtfluss, wobei sich der Gesamtfluss aus dem Aerosolfluss und/oder dem Luftfluss zusammensetzt. Ferner weist die Vorrichtung mindestens einen ersten Luftkanal zwischen dem Kompressor und der Vernebelungsrichtung, mindestens einen zweiten Luftkanal zwischen dem Kompressor und der Mischeinrichtung und mindestens eine Verstärkungseinrichtung zum Erhöhen des von dem Kompressor bereitgestellten Luftflusses auf.

Entsprechend einem weiteren Aspekt der Erfindung weist die Vorrichtung ferner oder anstelle der Verstärkungseinrichtung einen Bypasskanal auf, der den ersten mit dem zweiten Luftkanal verbindet und der dazu geeignet ist, den Luftfluss in den ersten Luftkanal unter Umgehung der Vernebelungseinrichtung in die Mischeinrichtung umzuleiten. Dieser Bypasskanal kann dabei vorzugsweise mit einem Ventil phasenweise zu- und weggeschaltet werden, wobei ein konstanter Gesamtfluss aufrechterhalten wird.

In einer bevorzugten Ausführungsform der Erfindung weist die Verstärkungseinrichtung mindestens eine Venturidüse auf. Diese ist vorzugsweise entlang des zweiten Luftkanals angeordnet und dazu geeignet, Umgebungsluft in den zweiten Luftkanal hinein anzusaugen, vorzugsweise über einen Filter. Die Venturidüse kann dabei bei einem Arbeitsdruck zwischen 0,5 und 5 bar, vorzugsweise zwischen 0,8 und 3 bar und besonders bevorzugt bei 1,2 bis 2 bar betrieben werden. Der dadurch bereitgestellte Luftfluss liegt in einem Bereich von 1 bis 60 Liter pro Minute. Der Kompressor und die Venturidüse sind dabei vorzugsweise so ausgelegt, dass keine Druck- und/oder Flussregelung notwendig ist.

In einer weiteren bevorzugten Ausführungsform sind zwei oder mehr Verstärkungseinrichtungen vorgesehen. Alternativ kann die Verstärkungsvorrichtung zwei oder mehr vorzugsweise in Reihe geschaltete Venturidüsen aufweisen. Dadurch soll insbesondere sichergestellt werden, dass auch dann ein hinreichender Fluss erreicht wird, wenn die Vernebelungseinrichtung nicht aktiv ist. Der Vorteil der in Reihe geschalteten Venturidüsen zeigt sich besonders bei kleinen Schlauchinnendurchmessern und Schläuchen mit einer Länge von mehr als 1 m. Schläuche mit kleinem Durchmesser und einer Länge von mehr als 1 m erleichtern die Handhabung des Verneblerhandgeräts. Schläuche, wie sie bei Beatmungsgeräten verwendet werden, werden von Patienten nicht akzeptiert und erhöhen das Kontaminationsrisiko, da aufgrund der großen Durchmesser Aerosoltröpfchen leichter in die Luftzuführung gelangen können. Bei der vorliegenden Erfindung kann ein Schlauch mit einer Länge von 0,2 m bis 2 m Durchmesser mit einem Innendurchmesser von 1 bis 20 mm verwendet werden, vorzugsweise wird ein Schlauchdurchmesser mit einem Innendurchmesser von 2 bis 5 mm verwendet mit einer Länge von 0,5 bis 1,5 m. Die Venturidüsen werden so dimensioniert, dass für ein exakt spezifiziertes Schlauchsystem mit bekanntem Durchflusswiderstand bei eingeschalteten und ausgeschalteten Vernebler exakt der gleiche Inspirationsfluss am Mundstück anliegt, ohne dass das Gerät ihn nachregeln muss.

Die Vorrichtung umfasst ferner bevorzugt eine Steuerungseinrichtung, die den Aerosolfluss und/oder den Luftfluss variieren bzw. vorgeben kann. Dabei soll erfindungsgemäß der Gesamtfluss aus Aerosol und/oder Luftfluss im Wesentlichen zeitlich konstant bleiben. Dieser liegt in einem Bereich zwischen 1 und 60 Liter pro Minute, vorzugsweise zwischen 3 und 50 Liter pro Minute. Die Vernebelungseinrichtung ist dazu geeignet, einen Aerosolfluss von 1 bis 20 Liter pro Minute, vorzugsweise von 3 bis 7 Liter pro Minute und besonders bevorzugt von etwa 6 Liter pro Minute zu erzeugen.

Ferner kann die Vorrichtung optional zumindest ein Rückschlagventil im zweiten Luftkanal aufweisen. Es ist außerdem daran gedacht, ein Entlüftungsventil zwischen Kompressor und Vernebelungseinrichtung einzubringen. Anstelle der Regelung mittels eines Entlüftungsventils ist es auch möglich, den Kompressor ein- bzw. auszuschalten.

Um eine Verwendung der Vorrichtung als Inhalationsgerät zu ermöglichen, ist es ferner vorgesehen, die Mischeinrichtung als Mundstück auszulegen.

Nachfolgend werden bevorzugte Ausführungsformen der erfindungsgemäßen Vorrichtung unter Bezugnahme auf die Figuren beispielhaft beschrieben. Es zeigen:
- Fig. 1: eine Prinzipskizze einer bevorzugten Ausführungsform des erfindungsgemäßen Inhalationsgerätes;
- Fig. 2a: eine Schaltskizze der Vorrichtung gemäß Fig. 1 mit den auftretenden Luftflüssen während der Inhalation mit eingeschaltetem Vernebler;
- Fig. 2b: eine Schaltskizze der Vorrichtung gemäß Fig. 1 mit den auftretenden Luftflüssen während der Inhalation bei abgeschaltetem Vernebler;
- Fig. 2c: eine Schaltskizze der Vorrichtung gemäß Fig. 1 mit den auftretenden Luftflüssen während der Exhalation;
- Fig.3 3: eine Prinzipskizze einer weiteren Ausführungsform des erfindungsgemäßen Inhalationsgerätes;
- Fig. 4: eine Prinzipskizze einer weiteren Ausführungsform des erfindungsgemäßen Inhalationsgerätes; und
- Fig. 5: eine Prinzipskizze einer weiteren Ausführungsform des erfindungsgemäßen Inhalationsgerätes.

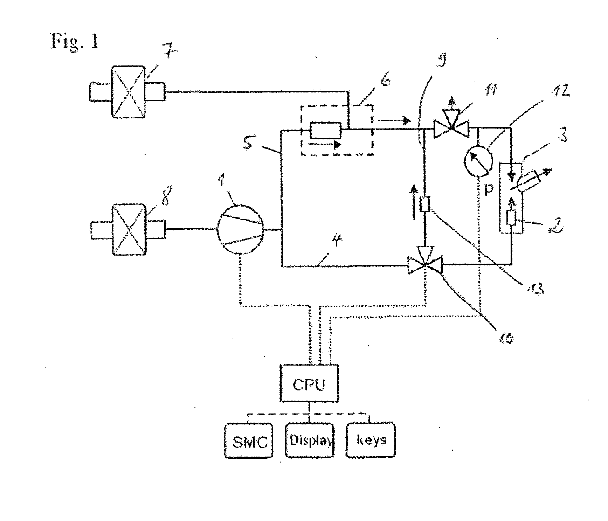

Fig. 1 zeigt schematisch eine bevorzugte Ausführungsform der erfindungsgemäßen Vorrichtung zum Bereitstellen eines Aerosolflusses und/oder eines Luftflusses. Die Vorrichtung umfasst einen Kompressor 1 zur Bereitstellung eines Luftflusses sowie eine Vernebelungseinrichtung 2 zum Erzeugen eines Aerosolflusses. Der Kompressor 1 und die Vernebelungseinrichtung 2 sind über einen ersten Luftkanal 4 miteinander verbunden. Die Vorrichtung weist ferner eine Mischeinrichtung 3 zum optionalen Vermischen des Aerosolflusses mit dem Luftfluss zu einem Gesamtfluss auf. In der bevorzugten Ausführungsform sind die Vernebelungseinrichtung 2 und die Mischeinrichtung 3 in einem Bauteil integriert. Es ist aber auch vorstellbar, dass die beiden Einrichtungen getrennt vorliegen und mittels eines Luftkanals miteinander verbunden sind. Um einen Gebrauch der Vorrichtung als Inhalationsgerät zu ermöglichen, ist in der dargestellten Ausführungsform die Mischeinrichtung mit einem Mundstück ausgebildet.

Die Mischeinrichtung 3 ist über einen zweiten Luftkanal 5 mit dem Kompressor 1 verbunden. Dadurch teilt sich der von dem Kompressor 1 bereitgestellte Luftfluss in zwei Teilflüsse auf, die durch die beiden Luftkanäle 4 und 5 strömen. Im zweiten Luftkanal 5 ist als Verstärkungseinrichtung eine Venturidüse 6 vorgesehen, die der Erhöhung des von dem Kompressor 1 bereitgestellten Luftflusses dient. Die Venturidüse 6 saugt zusätzliche Luft über den Filter 7 an, die dann über den zweiten Luftkanal 5 an die Mischeinrichtung 3 weitergeleitet wird. Alternativ zu der in Fig. 1 gezeigten Ausführungsform können auch zwei oder mehr Venturidüsen 6 vorgesehen sein, die vorzugsweise in Reihe geschaltet sind, um den bereitgestellten Luftfluss weiter zu verstärken.

Auch die Ansaugöffnung für den Kompressor 1 weist einen Luftfilter 8 auf. Die Verwendung einer Venturidüse ist vorteilhaft, da dann ein separates Überdruckventil nicht mehr erforderlich ist. Die Venturidüse ersetzt durch geeignete Geometriewahl das Überdruckventil. Ferner ist der Inhalationsfluss unabhängig von der Verneblergeometrie. Ist beispielsweise die Verneblerdüse zu klein (beispielsweise bei einem Austausch der Vernebler, wird dadurch der Fluß über den Vernebler geringer. Dies wird jedoch automatisch dadurch ausgeglichen, dass dann von dem vom Kompressor bereitgestellten Fluss mehr über die Venturidüse fließt. Der Kompressor ist dabei so ausgelegt, dass zusammen mit der Venturidüse und der Verneblerdüse ein idealer Arbeitspunkt erreicht wird und keine Regelung mehr erforderlich ist. Die entsprechenden Druckverhältnisse werden durch die geometrischen Verhältnisse der Düsen vorgegeben. Erfindungsgemäß bleibt der Inhalationsfluss immer gleich, lediglich das Inhalationsvolumen wird patientenabhängig eingestellt.

Das in Fig. 1 dargestellte Design ermöglicht gleichzeitig die Erzeugung eines hinreichend großen Druckes, um in der Vernebelungseinrichtung 2 mittels einer Vernebelungsdüse einen entsprechenden Wirkstoff zu zerstäuben, und das Aufrechterhalten eines hinreichend großen Luftflusses, der an das Atemminutenvolumen des Patienten angepasst ist. Dabei sorgt der Kompressor 1 für den nötigen Druck und die Venturidüse 6 für das hinreichende Flussvolumen. Diese Ausgestaltung der Inhalationsvorrichtung ist mit dem großen Vorteil verbunden, das ein Kompressor verwendet werden kann, der hinsichtlich seiner Leistung kleiner ausgelegt werden kann, als eigentlich zur Erzeugung der gewünschten Drücke und Flüsse notwendig wäre. Die Erfindung ermöglicht also eine bewusste Unterdimensionierung des Kompressors, was Kosten und Platzbedarf im Inhalationsgerätgehäuse spart.

Erfindungsgemäß weist die in Fig. 1 dargestellte bevorzugte Vorrichtung ferner einen Bypasskanal 9 auf, der mittels eines Bypassventils 10 zu- oder weggeschaltet werden kann. Der Bypasskanal dient zur Umgehung der Verneblungscinrichtung 2. Dem Bypasskanal 9 liegt die Idee zugrunde; einen konstanten Gesamtfluss, bereitzustellen, wobei die Verneblung ein- und ausgestellt werden kann. Wie weiter oben ausgeführt wurde, kann es vorteilhaft sein, die Vernebelung nur zeitweise während des Inhalationsvorganges auszuführen. Dadurch ist es möglich, zu kontrollieren, in welchem Bereich der Bronchien bzw. der Lungen das Wirkstoff-Aerosol eingeführt werden soll. Wird allerdings die Vernebelung während des Einatemvorganges unterbrochen, fließt also dann durch den ersten Luftkanal 4 keine Luft mehr, so soll der Patient nicht mit einem verminderten Luftfluss konfrontiert werden. Dies wird erfindungsgemäß gelöst, indem bei Deaktivierung der Vernebelungseinrichtung 2 das Bypassventil 10 so geschaltet wird, dass die durch den ersten Luftkanal 4 strömende Luft in den Bypasskanal 9 und den zweiten Luftkanal 5 umgeleitet wird. Dadurch wird weiterhin der gesamte durch den Kompressor 1 bereitgestellte Luftfluss in die Mischeinrichtung und damit dem Patienten zugeführt. Wird andererseits nicht der gesamte vom System bereitgestellte Luftfluss vom Patienten benötigt, beispielsweise während einer Inhalationspause, so kann überschüssige Luft durch das Ventil 11 abgegeben werden. Alternativ dazu lässt sich auch einfach der Kompressor 1 ein- bzw. ausschalten, um den Luftfluss zu regeln.

Die Steuerung bzw. Regelung der Ventile 10 und 11 sowie der Vernebelungseinrichtung 2 geschieht über eine Steuerungseinrichtung bzw. CPU, die mittels eines Drucksensors 12 die jeweilige Phase des Atemzyklus erfasst und die Ventile bzw. die Vernebelungseinrichtung entsprechend schaltet. Die Steuerungseinrichtung weist vorzugsweise ferner ein Display sowie Tasten zur Eingabe von Informationen durch den Benutzer auf.

Obwohl in der dargestellten Ausführungsform des Inhalatiansgerätes sowohl die Venturidüse 6 als auch der Bypasskanal 9 dargestellt sind, sind auch Ausführungsformen vorstellbar, in denen nur die Venturidüse 6 oder nur der Bypasskanal 9 vorhanden sind. Beide Merkmale führen jeweils für sich genommen dazu, dass mit einem leistungsschwächeren Kompressor 1 gearbeitet werden kann. In Kombination kann ein entsprechend stärkerer Effekt erzielt werden.

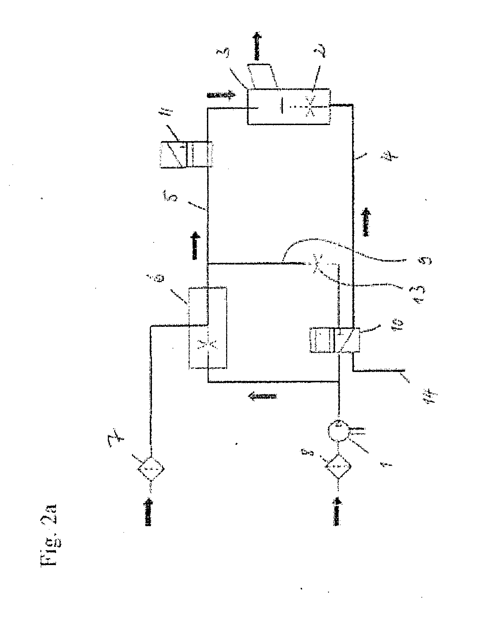

Fig. 2a zeigt eine Schaltskizze der Vorrichtung gemäß Fig. 1 und den Schaltzustand der Ventile 10 und 11 sowie die während der Inhalation mit eingeschaltetem Vernebler auftretenden Luftflüsse. Dabei ist das Ventil 10 so geschaltet, dass der Kompressor 1 mit der Vernebelungseinrichtung 2 in Verbindung steht und der Bypasskanal 9 blockiert. Ventil 11 ist gleichzeitig so geschaltet, dass der Kompressor mit der Mischeinrichtung 3 verbunden ist. Dies resultiert in den mit den Pfeilen dargestellten Luftwegen. Dementsprechend fließt Luft durch die Filter 7 und 8 in die Venturidüse und den Kompressor und wird über die Vernebelungseinrichtung 2 und die Mischeinrichtung 3 dem Patienten zur Verfügung gestellt. Der Bypasskanal 9 wird dabei nicht verwendet. Beispielsweise fließt über den ersten Luftkanal ein Fluss von 6 1/min und über die Venturidüse ein Fluß von 2,8 1/min bei beispielsweise 1,6 bar. Durch die Venturidüse wird der Fluss auf 9 1/min erhöht, dass in der Summe 15 1/min aus dem Verneblermundstüclk zum Patienten ausgegeben werden. Über die Venturidüsengeometrie wird der maximale Betriebsdruck eingestellt. Bei einer Inhalationspause würde der Bypasskanal zugeschaltet werden, so dass der Fluss von 6 1/min über den Bypasskanal zum zweiten Luftkanal 5 umgeleitet würde und in der Summe noch immer 15 1/min zum Patienten flössen.

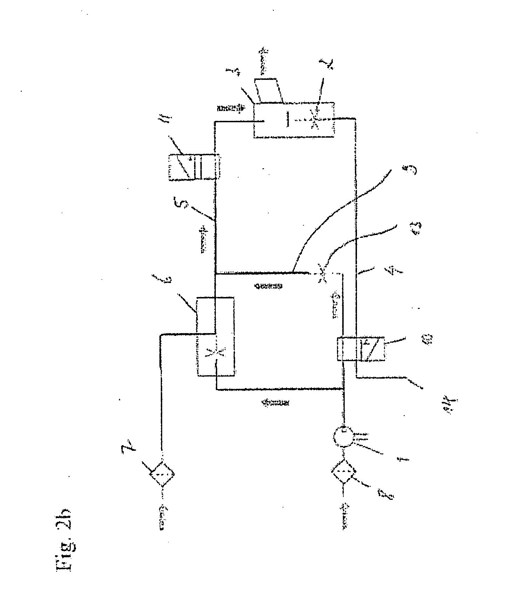

Fig. 2b zeigt eine entsprechende Schaltskizze für Inhalation mit abgeschalteter Vernebelungscinrichtung. Während das Ventil 11 wie in Schaltung der Fig. 2a geschaltet bleibt, ist nun das Ventil 10 auf Durchlass für den Bypasskanal 9 geschaltet. Entsprechend der Pfeile wird die vom Kompressor bereitgestellte Luft nun über den Bypasskanal 9 und den Luftkanal 5 in die Mischeinrichtung 3 geleitet und ausschließlich von letzterer dem Patienten zur Verfügung gestellt. Die Vernebelungseinrichtung ist dabei inaktiv. Um einen konstanten Gesamtfluss aufrecht zu erhalten, wird der durch den Bypasskanal 9 geleitete Luftstrom durch eine Ersatzdüse 13 geleitet, die dieselben Eigenschaften hat wie die Vernebelungsdüse in der Vernebelungseinrichtung 2. Gleichzeitig erlaubt die dargestellte Schaltung des Ventils 10 eine sofortige Entlüftung der Vernebelungseinrichtung 2 über einen Entlüftungskanal 14. Dadurch wird verhindert, dass ein im Luftkanal 4 verbliebener Restdruck ein weiteres Vernebeln des Wirkstoffes bewirken kann.

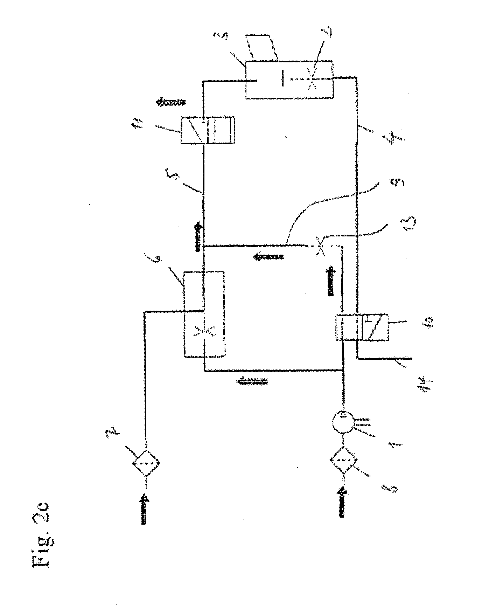

Fig. 2c zeigt eine entsprechende Schaltskizze für die Exhalation. Wieder stellen die Pfeile die entsprechenden Luftflüsse dar. Die Schaltung des Ventils 10 entspricht derjenigen der Fig. 2b. Dafür ist Ventil 11 nun so geschaltet, dass die vom Kompressor bereitgestellte Luft komplett durch eine Entlüftung entweichen kann. Gleichzeitig wird die Verbindung zwischen der Venturidüse bzw. dem Kompressor und der Mischeinrichtung 3 unterbunden, so dass ein Ausatmen in das Gerät verhindert wird.

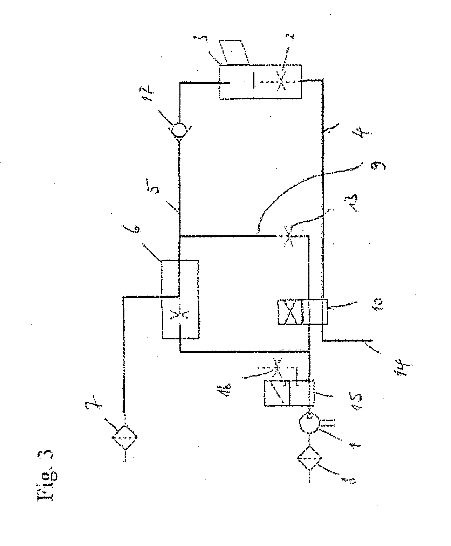

Fig. 3 zeigt eine alternative Ausführungsform der erfindungsgemäßen Vorrichtung, bei der das 3/2 Ventil, wie in Fig. 2 dargestellt, durch ein 4/2 Ventil ersetzt ist. Damit kann der Vernebler bei Umschaltung auf den Bypass sofort drucklos geschaltet werden. Ferner ist das Ventil 11 durch ein Rückschlagventil 17 ersetzt worden und ein zusätzliches Ventil 15 mit einem Entlüftungskanal 16 direkt hinter dem Kompressor 1 vorgesehen. Während des Ausatmungsvorganges verhindert nun das Rückschlagventil 17, dass in das Gerät ausgeatmet wird. Dies kann beispielsweise durch eine Silikonmembran geschehen. In dieser Phase des Atemzyklusses wird der Kompressor 1 über den Entlüftungskanal 16 entlüftet, wobei in diesem eine Düse eingebaut ist, um den Betriebsdruck des Kompressors zu halten.

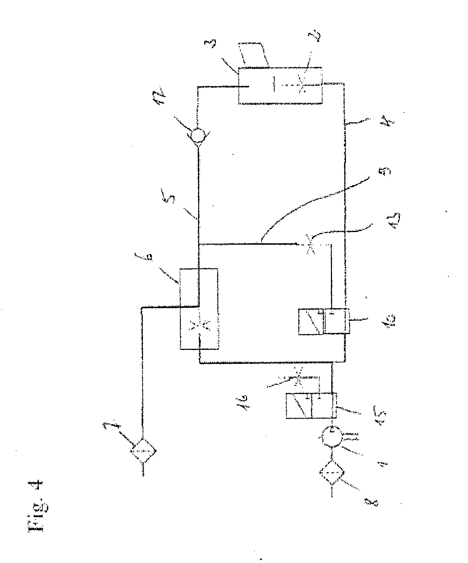

Fig. 4 zeigt eine weitere erfindungsgemäße Ausführungsform, bei der keine Entlüftung der Verneblerdüse vorgesehen ist. Wie bereits erwähnt, hat dies eine verzögerte Abschaltung der Aerosolerzeugung zur Folge, wodurch die Verabreichung des Medikaments weniger präzise gesteuert werden kann. Eine solche Ausführungsform kann jedoch aus Kostengründen dennoch in Betracht gezogen werden.

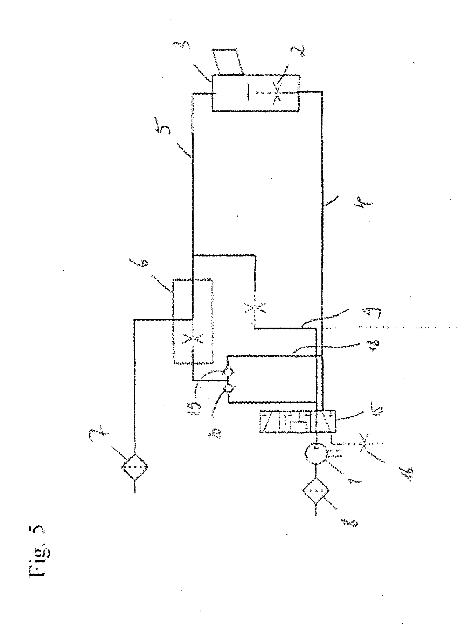

Eine besonders elegante Ausführungsform unter Verwendung nur eines einzigen Ventils 15 5 ist in Fig. 5 dargestellt. Die Entlüftung sowohl der Vernebelungseinrichtung als auch des Gesamtsystems während des Ausatmens kann hier über eine einzelne Entlüftungsleitung 16 geschehen. Hierfür ist allerdings ein dreistufig schaltbares 5/3 Ventil 15 notwendig. Ferner sind für eine geeignete Schaltung ein zusätzlicher Luftkanal 18 sowie zwei Rückschlagventile 19 und 20 einzubringen.

## Patentansprüche

1. Inhalationsgerät zur Bereitstellung eines Aerosolflusses und/oder eines Luftflusses mit:
a) mindestens einem Kompressor (1) zum Bereitstellen eines Luftflusses;
b) einer Vernebelungseinrichtung (2) zum Erzeugen eines Aerosolflusses;
c) einer Mischeinrichtung (3) zum optionalen Vermischen des Aerosolflusses mit dem Luftfluss zu einem Gesamtfluss, wobei sich der Gesamtfluss aus dem Aerosolfluss und/oder dem Luftfluss zusammensetzen kann;
d) mindestens einem ersten Luftkanal (4) zwischen dem Kompressor und der Vernebelungseinrichtung;
e) mindestens einem zweiten Luftkanal (5) zwischen dem Kompressor und der Mischeinrichtung;
f) einem Bypasskanal (9), der den ersten Luftkanal (4) mit dem zweiten Luftkanal (5) verbindet, der dazu geeignet ist, den Luftfluss in dem ersten Luftkanal (4) unter Umgehung der Vemebelungseinrichtung (2) in die Mischeinrichtung (3) umzuleiten; und
g) mindestens einer Verstärkungseinrichtung (6) zur Erhöhung des von dem Kompressor (1) bereitgestellten Luftflusses.

2. Vorrichtung nach Anspruch 1, wobei die Verstärkungseinrichtung (6) mindestens eine Venturidüse, vorzugsweise zwei Venturidüsen aufweist.

3. Vorrichtung nach Anspruch 2, wobei die Venturidüse entlang des zweiten Luftkanals (5) angeordnet ist und dazu geeignet ist, Umgebungsluft in den zweiten Luftkanal (5) hinein anzusaugen, vorzugsweise über einen Filter (7).

4. Vorrichtung nach Anspruch 2 oder 3, wobei die Venturidüse bei 0,5 bis 5 bar, vorzugsweise bei 0,8 bis 3 bar, besonders bevorzugt bei 1,2 bis 2 bar betrieben wird und einen Luftfluss von 1 bis 60 *l*/min bereitstellt.

5. Vorrichtung nach einem der vorangegangenen Ansprüche, ferner mit einer Steuerungseinrichtung, wobei der Aerosolfluss und/oder der Luftfluss von der Steuerungseinrichtung variiert bzw. vorgegeben werden können.

6. Vorrichtung nach einem der vorangegangenen Ansprüche, wobei der Gesamtfluss im Wesentlichen zeitlich konstant ist.

7. Vorrichtung nach einem der Ansprüche 1 bis 4, ferner mit einem Ventil (10), das ein phasenweises Zu- und Wegschalten des Bypasskanals (9) unter Aufrechterhaltung eines konstanten Gesamtflusses ermöglicht.

8. Vorrichtung nach einem der vorangegangenen Ansprüche, wobei der Kompressor (1) und die Einrichtung (6) zum Verstärken des Luftflusses so ausgelegt sind, dass keine Druck- und/oder Flussregelung notwendig ist.

9. Vorrichtung nach einem der vorangegangenen Ansprüche, wobei die Vernebelungseinrichtung (2) einen Aerosolfluss von 1 bis 20 l/min, vorzugsweise von 3 bis 7 *l*/min und besonders bevorzugt von etwa 6 *l*/min erzeugt.

10. Vorrichtung nach einem der vorangegangenen Ansprüche, wobei der bereitgestellte Gesamtfluss in einem Bereich von 1 bis 60 *l*/min, vorzugsweise bei etwa 3 bis 30 *l*/min liegt.

11. Vorrichtung nach einem der vorangegangenen Ansprüche, die ferner zumindest ein Rückschlagventil (17) im zweiten Luftkanal (5) aufweist.

12. Vorrichtung nach einem der vorangegangenen Ansprüche, die ferner zumindest ein Entlüftungsventil (10) zwischen Kompressor (1) und Vemebelungseinrichtung (2) aufweist.

13. Vorrichtung nach einem der vorangegangenen Ansprüche, bei dem die Mischeinrichtung (3) als Mundstück ausgelegt ist, um eine Verwendung der Vorrichtung als Inhalationsgerät zu ermöglichen.

14. Vorrichtung nach einem der vorangegangenen Ansprüche, die ferner ein Schlauchsystem zur Luftzuführung der Einatemluft zum Vernebler aufweist, das einen Schlauchinnendurchmesser von kleiner als 5 mm und eine Schlauchlänge von weniger als 1,5 m aufweist.

## Claims

1. An inhalation device for providing an aerosol flow and/or an air flow, comprising:
(a) at least a compressor (1) for providing an air flow;
(b) a nebulisation device (2) for generating an aerosol flow;
(c) a mixing means (3) for optionally mixing the aerosol flow with the air flow to form a total flow, wherein the total flow can consist of the aerosol flow and/or the air flow;
(d) at least a first air channel (4) arranged between the compressor and the nebulisation device;
(e) at least a second air channel (5) arranged between the compressor and the mixing means;
(f) a bypass channel (9) connecting the first air channel (4) with the second air channel (5), which is suitable for redirecting the air flow in the first air channel (4) into the mixing means (3) by bypassing the nebulisation device (2); and
(g) at least an amplification device (6) for increasing the air flow provided by the compressor (1).

2. The device according to claim 2, wherein the amplification means (6) comprises at least one Venturi nozzle, preferably two Venturi nozzles.

3. The device according to claim 1, wherein the Venturi nozzle is arranged along the second air channel (5) and suitable for sucking ambient air into the second air channel (5), preferably via a filter (7).

4. The device according to claim 2 or 3, wherein the Venturi nozzle is operated at 0.5 to 5 bar, preferably at 0.8 to 3 bar, particularly preferably at 1.2 to 2 bar and provides an air flow of 1 to 601/min.

5. The device according to any one of the preceding claims, further comprising a control means, wherein the aerosol flow and/or the air flow can be varied or determined by the control means.

6. The device according to any one of the preceding claims, wherein the total flow is substantially temporally constant.

7. The device according to any one of claims 1 to 4, further comprising a valve (10), which enables the activation or deactivation of the bypass channel (9) at certain intervals by maintaining a constant total flow.

8. The device according to any one of the preceding claims, wherein the compressor (1) and the means (6) for amplification of the air flow are designed such that no pressure and/or flow control is necessary.

9. The device according to any of the preceding claims, wherein the nebulisation device (2) generates an aerosol flow of 1 to 20 l/min, preferably of 3 to 7 *l*/min and particularly preferably of about 6 l/min.

10. The device according to any of the preceding claims, wherein the provided total flow is in a range between 1 to 60 l/min, preferably 3 to 30 l/min.

11. The device according to any one of the preceding claims, further comprising at least a check valve (17) in the second air channel (5).

12. The device according to any one of the preceding claims, further comprising at least a vent valve (10) between the compressor (1) and nebulisation device (2).

13. The device according to any one of the preceding claims, wherein the mixing means (3) is configured as mouthpiece to enable the use of the device as inhalation device.

14. The device according to any one of the preceding claims, further comprising a tube system for the air supply of the inhalation air to the nebulizer and having a tube inner diameter of less than 5 mm and a tube length of less than 1.5 m.

## Revendications

1. Appareil d'inhalation pour la préparation d'un flux d'aérosol et/ou d'un flux d'air, avec
a) au moins un compresseur (1) pour la préparation d'un flux d'air ;
b) un dispositif nébulisateur (2) pour la génération d'un flux d'aérosol ;
c) un dispositif mélangeur (3) pour le mélange facultatif du flux d'aérosol avec le flux d'air pour former un flux global, le flux global pouvant être composé du flux d'aérosol et/ou du flux d'air ;
d) au moins un premier canal d'air (4) entre le compresseur et le dispositif nébulisateur ;
e) au moins un deuxième canal d'air (5) entre le compresseur et le dispositif mélangeur ;
f) un canal de dérivation (9) qui relie le premier canal d'air (4) au deuxième canal d'air (5), prévu pour dériver le flux d'air du premier canal d'air (4) vers le dispositif mélangeur (3) en contournant le dispositif nébulisateur (2) ; et
g) au moins un dispositif amplificateur (6) pour accroître le flux d'air préparé par le compresseur.

2. Appareil selon la revendication 1, où le dispositif amplificateur (6) comporte au moins un tube de Venturi, préférentiellement deux tubes de Venturi.

3. Appareil selon la revendication 2, où le tube de Venturi est disposé le long du deuxième canal d'air (5) et est prévu pour pomper l'air ambiant dans le deuxième canal d'air (5), préférentiellement par l'intermédiaire d'un filtre (7).

4. Appareil selon la revendication 2 ou la revendication 3, où le tube de Venturi est mis en service sous 0,5 à 5 bar, avantageusement sous 0,8 à 3 bar, préférentiellement sous 1,2 à 2 bar, et prépare un flux d'air compris entre 1 et 60 l/min.

5. Appareil selon l'une des revendications précédentes, comportant en outre un dispositif de commande, où le flux d'aérosol et/ou le flux d'air peuvent être modifiés ou prescrits par le dispositif de commande.

6. Appareil selon l'une des revendications précédentes, où le flux global est sensiblement constant dans le temps.

7. Appareil selon l'une des revendications 1 à 4, comportant en outre une vanne (10) permettant une activation et une désactivation graduelles du canal de dérivation (9) en maintenant un flux global constant.

8. Appareil selon l'une des revendications précédentes, où le compresseur (1) et le dispositif (6) pour l'accroissement du flux d'air sont prévus pour rendre superflue une régulation de pression et/ou de débit.

9. Appareil selon l'une des revendications précédentes, où le dispositif nébulisateur (2) génère un flux d'aérosol compris entre 1 et 20 l/min, avantageusement entre 3 et 7 l/min et préférentiellement sensiblement égal à 6 l/min.

10. Appareil selon l'une des revendications précédentes, où le flux global préparé est compris entre 1 et 60 l/min, préférentiellement sensiblement entre 3 et 30 l/min.

11. Appareil selon l'une des revendications précédentes, comportant en outre au moins une vanne anti-retour (17) dans le deuxième canal d'air (5).

12. Appareil selon l'une des revendications précédentes, comportant en outre au moins une vanne de purge d'air (10) entre le compresseur (1) et le dispositif nébulisateur (2).

13. Appareil selon l'une des revendications précédentes, où le dispositif mélangeur (3) est prévu comme embout pour permettre une utilisation de l'appareil comme appareil d'inhalation.

14. Appareil selon l'une des revendications précédentes, comportant en outre un système de tuyau flexible pour la conduction d'air de l'air inhalé au nébulisateur, lequel présente un diamètre intérieur de tuyau inférieur à 5 mm et une longueur de tuyau inférieure à 1,5 m.
